# EUROPEAN PATENT APPLICATION

(11) **EP 4 249 123 A1**
(43) Date of publication of application: **27.09.2023**
(21) Application number: 22179719.4
(22) Date of filing: 17.06.2022
(51) Int. Cl.: B01L 7/00, B01L 9/06, B01L 3/00

(54) **MULTI-FUNCTION POLYMERASE CHAIN REACTION DEVICE AND CONTROLLING METHOD THEREOF**

(30) Priority: 22.03.2022 TW 111110534
(71) Applicant: Labturbo Biotech Corporation, Taipei City 111052 (TW)
(72) Inventor: CHI-SHENG, Tai, Taipei (TW); SHIH-JENG, Dai, Taipei (TW); BO CHEN, Lin, Taipei (TW)
(74) Representative: Sach, Greg Robert

(57) **Abstract**

The present invention discloses a multi-function polymerase chain reaction device, which utilizes a movable multi-functional top cover to make an equipment can selectively perform variable temperature PCR (Polymerase Chain Reaction) or constant temperature PCR, which the flexibility of test-application is highly raised.

## Description

### FIELD OF THE INVENTION

The present invention relates to a multi-function polymerase chain reaction (PCR) device and controlling method thereof, and, is related to bio-technology industry, for applying in automatic devices.

### BACKGROUND OF THE INVENTION

In the field of bio-technology, for DNA and RNA, generally, there are 2 experimental tasks: 1. Qualitative; 2. Quantitative. Both tasks are usually using Polymerase Chain Reaction (PCR). Meanwhile, the most basic principle of PCR is to raise temperature for denaturation; then decrease temperature & slow cooling for annealing; then raise temperature for extension; then repeat the 3 steps (raise -> decrease -> raise).

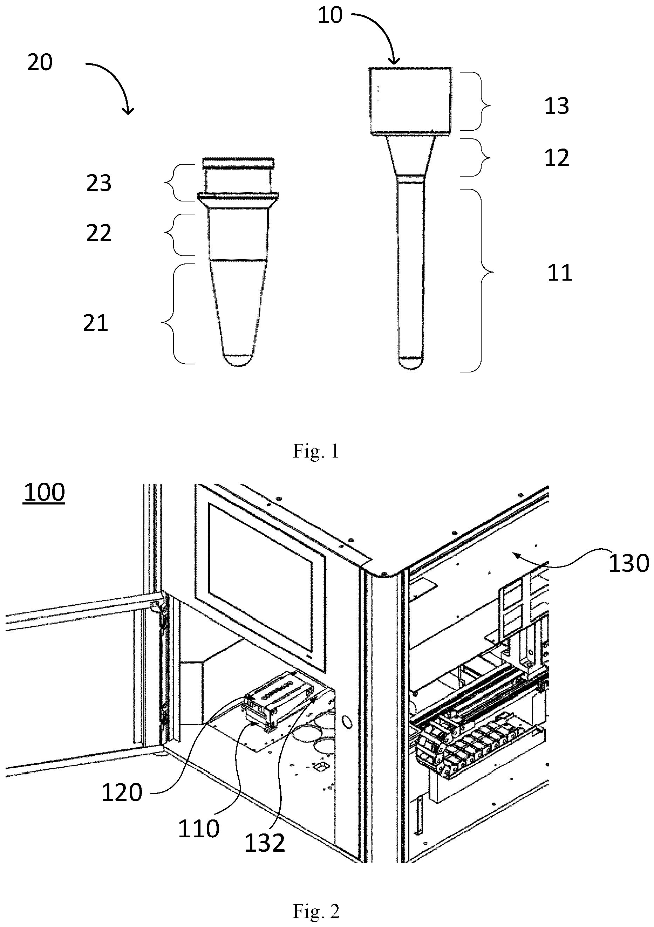

With technology development, there are many methods are developed for qualitative and quantitative. With reference of Fig. 1, which illustrates PCR tube 10 and PCR capillary tube 20. For qualitative PCR, the most common method is capillary convective PCR (CCPCR), which adopts the PCR capillary tube 10 in the right side of the Fig. 1 and perform PCR with temperature-stable (the temperature is keep in a certain range which is different from the basic PCR). The tube 10 comprises a capillary portion 11 (lower and thinner part), a neck portion 12 and a cap portion 13. The capillary portion 11 can be called as reaction region, which the liquid for PCR reaction is reacted in this region. For quantitative PCR, the most common method is quantitative PCR (qPCR), which adopts the PCR tube 20 in the left side of the Fig. 1 and perform PCR with temperature-variable (the same as the basic PCR). The tube 20 comprises a bottom portion 21, a neck portion 22 and a cap portion 23. The bottom portion 21 can be called as reaction region, which the liquid for PCR reaction is reacted in this region. Please noted that the bottom portion 21 of the tube 20 is cone-shaped, which is for demonstrated for differing from the tube 10, for the tube 20 has no capillary portion 11.

However, in the conventional art, the heating method and shapes of tubes are different in CCPCR and qPCR, two different equipment are needed to respectively perform CCPCR and qPCR. The setup cost is raised and impossible to adjust task according to different needs (qualitative or quantitative).

Hence, it is needed to provide a multi-function polymerase chain reaction (PCR) device and controlling method thereof, for solving the technical problem.

### SUMMARY OF THE INVENTION

In order to solve the technical problem, the present invention provides a multi-function polymerase chain reaction (PCR) device, which utilizes a movable multifunctional upper cover to make one and the same equipment can perform temperature-variable PCR (such as qPCR) or temperature-stable PCR (such as CCPCR), then the flexibility of application of one and the same equipment is hugely raised.

In order to solve the technical problems of the conventional art, the object of the present invention is to provide a multi-function polymerase chain reaction device, which comprises a multifunctional upper cover, a lower carrier, and a driving device. The lower carrier is disposed below the multifunctional upper cover and for storing at least one reaction tube. The driving device is used to selectively move the multifunctional upper cover onto the lower carrier before or after the at least one reaction tube is disposed in the lower carrier.

In one preferred embodiment, the lower carrier further comprises a first heating unit, which touches and controls a first position of the at least one reaction tube at a first temperature range.

In one preferred embodiment, the multifunctional upper cover further comprises a second heating unit, which touches and controls a second position respectively to the first position of the at least one reaction tube at a second temperature range.

In one preferred embodiment, the driving device determines a sequence of moving the multifunctional upper cover and the at least one reaction tube according to whether multi-function polymerase chain reaction device performs temperature-variable polymerase chain reaction or temperature-stable polymerase chain reaction.

In one preferred embodiment, the at least one reaction tube is chosen as PCR tube or PCR capillary tube according to whether multi-function polymerase chain reaction device performs temperature-variable polymerase chain reaction or temperature-stable polymerase chain reaction.

In one preferred embodiment, when the multifunctional upper cover is moved onto the lower carrier after the at least one reaction tube is disposed in the lower carrier, a first heating unit of the lower carrier controls a first position of the at least one reaction tube at a first temperature range and a second heating unit of the multifunctional upper cover controls a top cover area of the at least one reaction tube at a second temperature range.

In order to solve the technical problems of the conventional art, the object of the present invention is to further provide an operational method for a multi-function polymerase chain reaction device, which comprises: first, an external information is received to determine whether temperature-variable polymerase chain reaction or temperature-stable polymerase chain reaction is going to perform. Then, a multifunctional upper cover is electively moved onto the lower carrier before or after at least one reaction tube is placed in the lower carrier.

In one preferred embodiment, the operational method further comprises: when temperature-variable polymerase chain reaction is performed, the driving device moves the multifunctional upper cover onto the lower carrier after the at least one reaction tube is disposed in the lower carrier, a first heating unit of the lower carrier controls a first position of the at least one reaction tube at a first temperature range and a second heating unit of the multifunctional upper cover controls a top cover area of the at least one reaction tube at a second temperature range.

In one preferred embodiment, the operational method further comprises: when temperature-stable polymerase chain reaction is performed, the driving device moves the multifunctional upper cover onto the lower carrier before the at least one reaction tube is disposed in the lower carrier, the at least one reaction tube is passing through the multifunctional upper cover and the lower carrier and touches a first heating unit of the lower carrier to control a first position of the at least one reaction tube at a first temperature range.

In one preferred embodiment, the operational method further comprises: a second heating unit of the multifunctional upper cover is used to touch and control a second position with respect to the first position of the at least one reaction tube at a second temperature range.

Compared with the conventional arts, the present invention utilizes a movable multifunctional upper cover to make one and the same equipment can perform temperature-variable PCR (such as qPCR) or temperature-stable PCR (such as CCPCR), then the flexibility of application of one and the same equipment is hugely raised.

### DESCRIPTION OF THE DIAGRAMS

Fig. 1 is a schematic diagram of PCR tube and PCR capillary tube;
Fig. 2 is a whole schematic diagram of the multi-function polymerase chain reaction (PCR) device according to the present invention;
Fig. 3 is a partial-transparent side schematic diagram of the multi-function polymerase chain reaction (PCR) device according to first preferred embodiment of the present invention;
Fig. 4 is a partial-transparent side schematic diagram of the multi-function polymerase chain reaction (PCR) device according to second preferred embodiment of the present invention;
Fig. 5 is a flow diagram of an operational method for a multi-function polymerase chain reaction device according to first preferred embodiment of the present invention;
Figs. 6A- 6C are kinematic diagrams of the multi-function polymerase chain reaction (PCR) device according to first preferred embodiment of the present invention;
Fig. 7 is a flow diagram of an operational method for a multi-function polymerase chain reaction device according to second preferred embodiment of the present invention; and
Figs. 8A- 8C are kinematic diagrams of the multi-function polymerase chain reaction (PCR) device according to second preferred embodiment of the present invention.

### DETAILED DESCRIPTION OF THE PREFERRED EMBODIMENTS

The following description of the embodiments is given by way of illustration with reference to the specific embodiments in which the invention may be practiced. The terms such as "up", "down", "front", "back", "left", "right", "inside", "outside", "side", etc., The direction of the diagram. Accordingly, the use of a directional term is used to describe and to understand the present invention and is not intended to limit the invention.

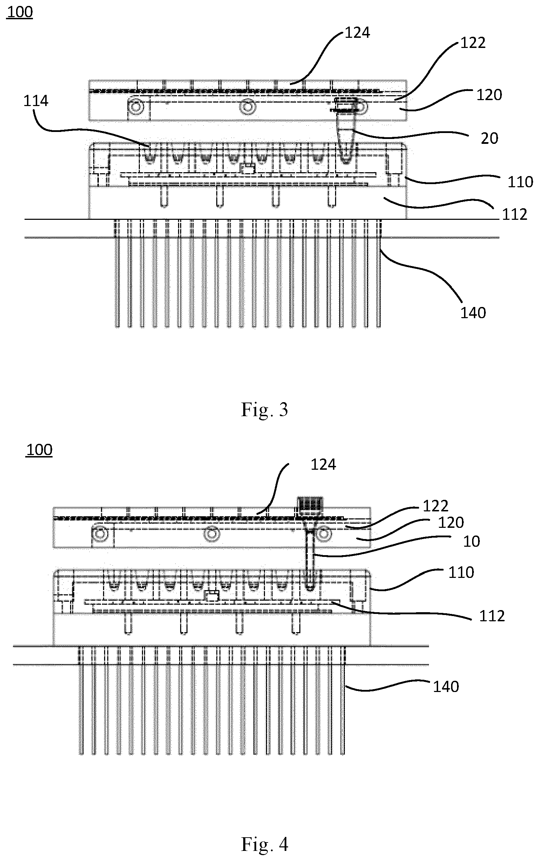

Please refer to Fig. 2, which is a whole schematic diagram of the multi-function polymerase chain reaction (PCR) device 100 according to the present invention. The multi-function polymerase chain reaction device 100 comprises a multifunctional upper cover 120, a lower carrier 110 and a driving device 130. The lower carrier 110 is disposed below the multifunctional upper cover 120 and for storing at least one reaction tube (not shown, 20 in Fig. 3 or 10 in Fig. 4). In detail, "below" means a configuration in vertical direction, which will be easier understand in Figs. 3-4. Moreover, in order to avoid misleading, the at least one reaction tube will be described in Figs. 3-4. The driving device 130 is used to selectively move the multifunctional upper cover 120 onto the lower carrier 110 before or after the at least one reaction tube is disposed in the lower carrier 110. It should be noted that the present invention only focuses on the movement relationship between the multifunctional upper cover 120 and the lower carrier 110 (affecting the relative positions of the multifunctional upper cover 120, the lower carrier 110 and the at least one reaction tube), and the driving device 130 can use the sliding rod 132 as shown in the figure to move the multifunctional upper cover 120, but other methods can also be used, which is not limited to this.

Please refer to Fig. 3, which is a partial-transparent side schematic diagram of the multi-function polymerase chain reaction (PCR) device 100 according to first preferred embodiment of the present invention. This figure is a schematic diagram of a reaction tube 20 (refer to FIG. 1 and related descriptions). In this preferred embodiment, because the multifunctional polymerase chain reaction device 100 performs the temperature-variable polymerase chain reaction, the at least one reaction tube 20 is selected as PCR tube, so the relative relationship from bottom to top is: the lower carrier 110, the at least one reaction tube 20 and the multifunctional upper cover 120. The lower carrier 110 also comprises a first heating unit 112 and at least one groove 114, and the first heating unit 112 contacts and controls the first position of the at least one reaction tube 20 (referring to the bottom portion 21 in Fig. 1) through the at least one groove 114 in a first temperature range. In this preferred embodiment, the first heating unit 112 is heated by solid contact, but it is not limited to this, and it may also be liquid, gas contact, or even through magnetic force and other non-contact forces. At the same time, the first heating unit 112 is also in contact with a heat dissipation unit 140. The heat dissipation unit 140 is used to provide a cooling effect. In this preferred embodiment, the heat dissipation unit 140 is a fin-shaped aluminum, but not limited thereto.

Further, the multifunctional upper cover 120 further comprises at least one through hole 124 corresponding to the at least one reaction tube 20. When the at least one reaction tube 20 is a PCR tube, and the reaction area of the PCR tube (Refer to Fig. 1) is not allowed to pass from above the at least through hole 124. This design is to avoid misuse of PCR tubes.

Therefore, when the multifunctional upper cover 120 is moved onto the lower carrier 110 after the at least one reaction tube 20 is disposed in the lower carrier 110, a first heating unit 114 of the lower carrier 110 controls a first position of the at least one reaction tube 20 (referring to the bottom portion 21 in Fig. 1) at a first temperature range and a second heating unit 122 of the multifunctional upper cover 120 controls a top cover area of the at least one reaction tube 20 (referring to the cap portion 23) at a second temperature range. The reason is that, in general, the first temperature range of the temperature-variable PCR is usually greater than 90 Celsius degrees C. Therefore, it is necessary to control the temperature of the lid area of the reaction tube to increase to a certain extent to avoid explosion due to excessive gas pressure inside the reaction tube.

Please refer to Fig. 4, which is a partial-transparent side schematic diagram of the multi-function polymerase chain reaction (PCR) device 100 according to second preferred embodiment of the present invention. The difference between the present embodiment and the first preferred embodiment is: because the multifunctional polymerase chain reaction device 100 performs the temperature-stable polymerase chain reaction, the at least one reaction tube 10 is selected as PCR capillary tube, so the relative relationship from bottom to top is: the lower carrier 110, the multifunctional upper cover 120 and the at least one reaction tube 10.

When the at least one reaction tube 10 is PCR capillary tube, the reaction region (referring to Fig. 1) of the at least one reaction tube 10 is allowed to pass through the at least one through hole 124. Meanwhile, the multifunctional upper cover 120 further comprises a second heating unit 122, which touches and controls a second position (upper portion of the capillary portion 11 in Fig. 1) respectively to the first position of the at least one reaction tube 10 at a second temperature range, then the at least one reaction tube can be performed with the temperature-stable PCR. The value of the first temperature range is higher than the value of the second temperature range. In a preferred embodiment, with the second heating unit 122 and the first heating unit 112, it has more accurate temperature control than the existing single heat source, and can better perform PCR operations.

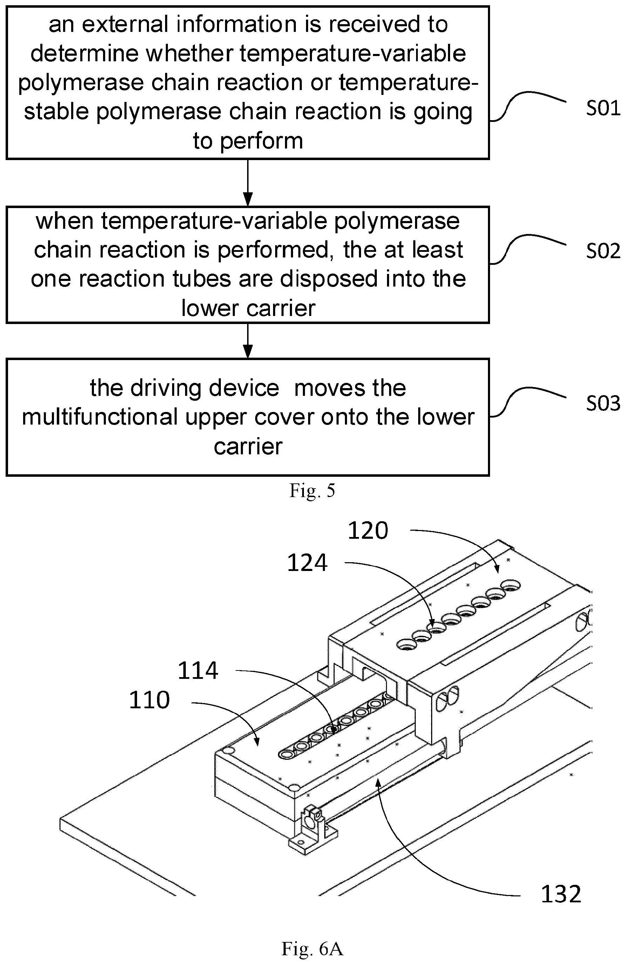

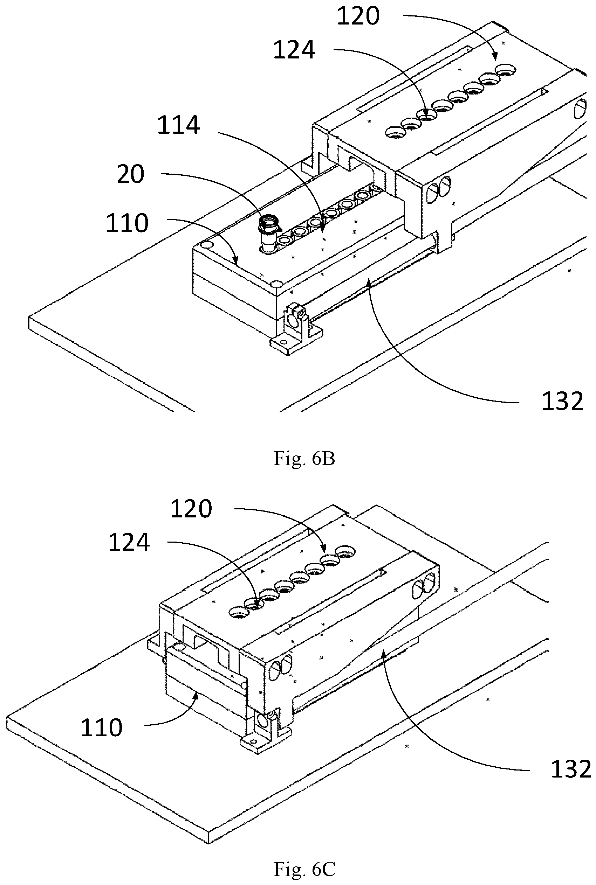

Please refer to Figs. 5-6C, Fig. 5 is a flow diagram of an operational method for a multi-function polymerase chain reaction device 100 according to first preferred embodiment of the present invention; Figs. 6A- 6C are kinematic diagrams of the multi-function polymerase chain reaction (PCR) device according to first preferred embodiment of the present invention. The elements mentioned in the flow diagram, please refer to the Figs 1-4 and the relative description. first, the step S01, an external information is received to determine whether temperature-variable polymerase chain reaction or temperature-stable polymerase chain reaction is going to perform, which means the Fig. 6A. Then, step S02, when temperature-variable polymerase chain reaction is performed, the at least one reaction tubes 20 are disposed into the lower carrier 110, which means Fig. 6B. Then, step S03, the driving device 130 moves the multifunctional upper cover 120 onto the lower carrier 120, which means Fig. 6C. In detail, the first heating unit 112 of the lower carrier 110 controls a first position (referring to bottom portion 21 in Fig. 1) of the at least one reaction tube 20 at a first temperature range and a second heating unit 122 of the multifunctional upper cover 120 controls a top cover area (referring to cap portion 23 in Fig. 1) of the at least one reaction tube 20 at a second temperature range. In the real practice, after temperature-variable polymerase chain reaction or temperature-stable polymerase chain reaction is chosen, with placing an order to the equipment, the multi-function polymerase chain reaction device 100 will orderly move the at least one reaction tube 20 and the multifunctional upper cover 120, then begin to perform the corresponding PCR process. The technique to move the at least one reaction tube 20 and the multifunctional upper cover 120 can be referred to any conventional art, not limited by the embodiments.

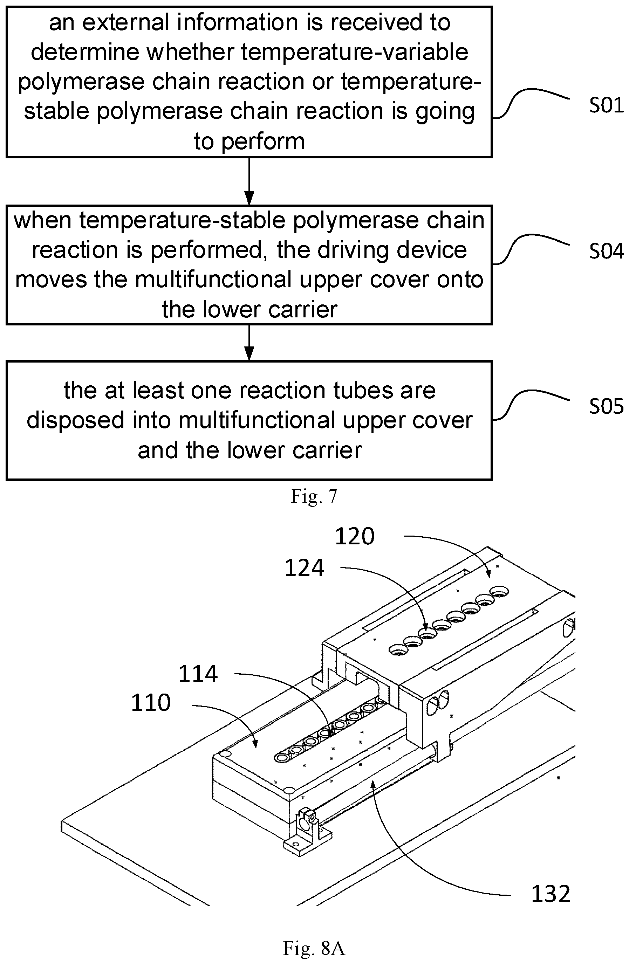

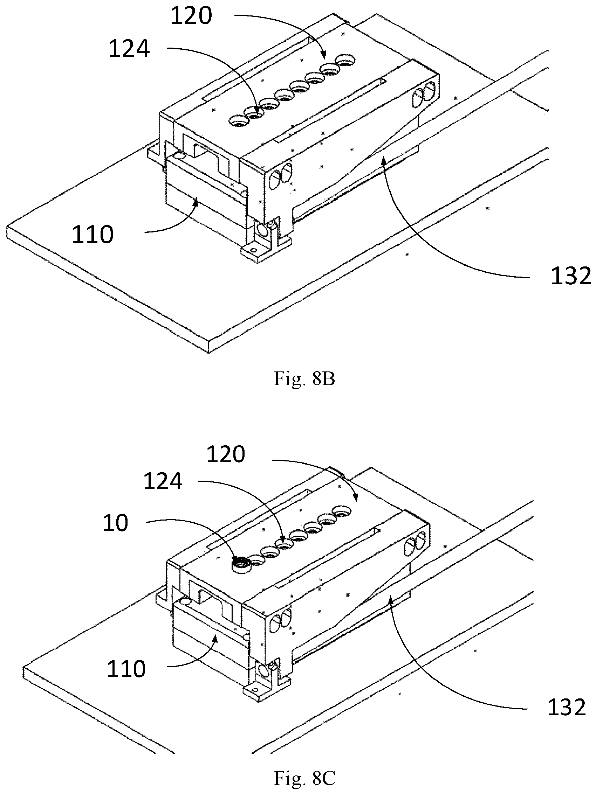

Please refer to Figs. 7-8C, Fig. 7 is a flow diagram of an operational method for a multi-function polymerase chain reaction device 100 according to second preferred embodiment of the present invention; Figs. 8A- 8C are kinematic diagrams of the multi-function polymerase chain reaction (PCR) device according to second preferred embodiment of the present invention. The elements mentioned in the flow diagram, please refer to the Figs 1-4 and the relative description. first, the step S01, an external information is received to determine whether temperature-variable polymerase chain reaction or temperature-stable polymerase chain reaction is going to perform, which means the Fig. 8A (the same situation as Fig. 6A). Then, step S04, when temperature-stable polymerase chain reaction is performed, the driving device 130 moves the multifunctional upper cover 120 onto the lower carrier 110, which means Fig. 8B. Then, step S05, the at least one reaction tubes 10 are disposed into multifunctional upper cover 120 and the lower carrier 110, the reaction region (referring to Fig. 1) of the at least one reaction tube 10 is passing through the multifunctional upper cover 120 and the lower carrier 110 and touches a first heating unit 112 of the lower carrier 110 to control a first position of the at least one reaction tube 10 at a first temperature range, which means Fig. 8C. In the real practice, after temperature-variable polymerase chain reaction or temperature-stable polymerase chain reaction is chosen, with placing an order to the equipment, the multi-function polymerase chain reaction device 100 will orderly move the at least one reaction tube 20 and the multifunctional upper cover 120, then begin to perform the corresponding PCR process. The technique to move the at least one reaction tube 20 and the multifunctional upper cover 120 can be referred to any conventional art, not limited by the embodiments.

Compared with the conventional art, the present invention utilizes a movable multifunctional upper cover to make one and the same equipment can perform temperature-variable PCR or temperature-stable PCR, then the flexibility of application of one and the same equipment is hugely raised.

As described above, although the present invention comprises been described with the preferred embodiments thereof, those skilled in the art will appreciate that various modifications, additions, and substitutions are possible without departing from the scope and the spirit of the invention. Accordingly, the scope of the present invention is intended to be defined only by reference to the claims.

## Claims

1. A multi-function polymerase chain reaction device, comprising:
a multifunctional upper cover;
a lower carrier, disposing below the multifunctional upper cover and for storing at least one reaction tube; and
a driving device, selectively moving the multifunctional upper cover onto the lower carrier before or after the at least one reaction tube is disposed in the lower carrier.

2. The multi-function polymerase chain reaction device according to claim 1, wherein the lower carrier further comprises a first heating unit, which touches and controls a first position of the at least one reaction tube at a first temperature range.

3. The multi-function polymerase chain reaction device according to claim 2, wherein the multifunctional upper cover further comprises a second heating unit, which touches and controls a second position respectively to the first position of the at least one reaction tube at a second temperature range.

4. The multi-function polymerase chain reaction device according to claim 1, wherein the driving device determines a sequence of moving the multifunctional upper cover and the at least one reaction tube according to whether multi-function polymerase chain reaction device performs temperature-variable polymerase chain reaction or temperature-stable polymerase chain reaction.

5. The multi-function polymerase chain reaction device according to claim 1, wherein the at least one reaction tube is chosen as PCR tube or PCR capillary tube according to whether multi-function polymerase chain reaction device performs temperature-variable polymerase chain reaction or temperature-stable polymerase chain reaction.

6. The multi-function polymerase chain reaction device according to claim 1, wherein when the multifunctional upper cover is moved onto the lower carrier after the at least one reaction tube is disposed in the lower carrier, a first heating unit of the lower carrier controls a first position of the at least one reaction tube at a first temperature range and a second heating unit of the multifunctional upper cover controls a top cover area of the at least one reaction tube at a second temperature range.

7. An operational method for a multi-function polymerase chain reaction device, which comprises:
receiving an external information to determine whether temperature-variable polymerase chain reaction or temperature-stable polymerase chain reaction is going to perform;
selectively moving a multifunctional upper cover onto the lower carrier before or after at least one reaction tube is placed in the lower carrier.

8. The operational method for a multi-function polymerase chain reaction device according to claim 7, which further comprises:
when temperature-variable polymerase chain reaction is performed, the driving device moves the multifunctional upper cover onto the lower carrier after the at least one reaction tube is disposed in the lower carrier, a first heating unit of the lower carrier controls a first position of the at least one reaction tube at a first temperature range and a second heating unit of the multifunctional upper cover controls a top cover area of the at least one reaction tube at a second temperature range.

9. The operational method for a multi-function polymerase chain reaction device according to claim 7, which further comprises:
when temperature-stable polymerase chain reaction is performed, the driving device moves the multifunctional upper cover onto the lower carrier before the at least one reaction tube is disposed in the lower carrier, the at least one reaction tube is passing through the multifunctional upper cover and the lower carrier and touches a first heating unit of the lower carrier to control a first position of the at least one reaction tube at a first temperature range.

10. The operational method for a multi-function polymerase chain reaction device according to claim 9, which further comprises:
a second heating unit of the multifunctional upper cover is used to touch and control a second position with respect to the first position of the at least one reaction tube at a second temperature range.

## Amended claims

### Amended claims in accordance with Rule 137(2) EPC.

1. A multi-function polymerase chain reaction device (100), **characterized in** comprising:
a multifunctional upper cover (120) comprising at least one through hole (124);
a lower carrier (110), disposing below the multifunctional upper cover (120) and for storing at least one reaction tube (10, 20); and
a driving device (130), moving the multifunctional upper cover (120) onto the lower carrier (110) before the at least one reaction tube (10, 20) is manually disposed in the lower carrier (110) by inserting through the at least one through hole (124) when the at least one reaction tube (10, 20) is a PCR capillary tube (10) or moving the multifunctional upper cover (120) onto the lower carrier (110) after the at least one reaction tube (10, 20) is manually disposed in the lower carrier (110) when the at least one reaction tube (10, 20) is a PCR tube (20).

2. The multi-function polymerase chain reaction device (100) according to claim 1, **characterized in that** the lower carrier (110) further comprises a first heating unit (112), which heats a first position of the at least one reaction tube (10, 20) via contact with each other in a first temperature range.

3. The multi-function polymerase chain reaction device (100) according to claim 2, **characterized in that** the multifunctional upper cover (120) further comprises a second heating unit (122), which heats a second position with respect to the first position of the at least one reaction tube (10, 20) via contact with each other in a second temperature range.

4. The multi-function polymerase chain reaction device (100) according to claim 1, **characterized in that** the driving device (130) moves the multifunctional upper cover (120) before or after the at least one reaction tube (10, 20) is manually disposed in the lower carrier (110) according to whether the multi-function polymerase chain reaction device (100) performs a temperature-variable polymerase chain reaction or a temperature-stable polymerase chain reaction by receiving an external information.

5. The multi-function polymerase chain reaction device (100) according to claim 1, **characterized in that** when the multifunctional upper cover (120) is moved onto the lower carrier (110) after the at least one reaction tube (10, 20) is disposed in the lower carrier (110), a first heating unit (112) of the lower carrier (110) controls a first position of the at least one reaction tube (10, 20) in a first temperature range and a second heating unit (122) of the multifunctional upper cover (120) controls a top cover area of the at least one reaction tube (10, 20) in a second temperature range.

6. An operational method for a multi-function polymerase chain reaction device, **characterized in** comprising:
receiving an external information to perform a temperature-variable polymerase chain reaction or a temperature-stable polymerase chain reaction;
moving a multifunctional upper cover (120) having at least one through hole (124) onto the lower carrier (110) before at least one reaction tube (10, 20) is manually disposed in the lower carrier (110) by inserting through the at least one through hole (124) when the at least one reaction tube (10, 20) is a PCR capillary tube (10) or moving the multifunctional upper cover (120) onto the lower carrier (110) after the at least one reaction tube (10, 20) is manually disposed in the lower carrier (110) when the at least one reaction tube (10, 20) is a PCR tube (20).

7. The operational method for a multi-function polymerase chain reaction device according to claim 6, **characterized in that** the operational method further comprises:
when a temperature-variable polymerase chain reaction is performed, the driving device (130) moves the multifunctional upper cover (120) onto the lower carrier (110) after the at least one reaction tube (10, 20) is manually disposed in the lower carrier (110), a first heating unit (112) of the lower carrier (110) controls a first position of the at least one reaction tube (10, 20) in a first temperature range and a second heating unit (122) of the multifunctional upper cover (120) controls a top cover area of the at least one reaction tube (10, 20) in a second temperature range.

8. The operational method for a multi-function polymerase chain reaction device according to claim 6, **characterized in that** the operational method further comprises:
when a temperature-stable polymerase chain reaction is performed, the driving device (130) moves the multifunctional upper cover (120) onto the lower carrier (110) before the at least one reaction tube (10, 20) is manually disposed in the lower carrier (110), the at least one reaction tube is inserted through the at least one through hole (124) of the multifunctional upper cover (120) and in the lower carrier (110), and a first heating unit (112) of the lower carrier (110) heats a first position of the at least one reaction tube (10, 20) via contact with each other in a first temperature range.

9. The operational method for a multi-function polymerase chain reaction device according to claim 8, **characterized in that** the operational method further comprises:
a second heating unit (122) of the multifunctional upper cover (120) heats a second position with respect to the first position of the at least one reaction tube (10, 20) via contact with each other in a second temperature range.
